# EUROPÄISCHE PATENTSCHRIFT

(11) **EP 0 949 901 B1**
(45) Veröffentlichungstag und Bekanntmachung des Hinweises auf die Patenterteilung: **07.11.2001**
(21) Anmeldenummer: 97953636.4
(22) Anmeldetag: 12.12.1997
(51) Int. Cl.: A61K 7/48

(54) **KOSMETISCHES REINIGUNGS- UND PFLEGEPRÄPARAT ENTHALTEND EXTRAKTE VON PFLANZEN UND ALGEN**
COSMETIC CLEANSING AND SKIN CARE PREPARATION CONTAINING PLANT AND ALGAE EXTRACTS
PREPARATION COSMETIQUE DE SOINS ET DE TOILETTE CONTENANT DES EXTRAITS DE PLANTES ET D'ALGUES

(30) Priorität: 18.12.1996 DE 1965450
(43) Veröffentlichungstag der Anmeldung: 20.10.1999
(73) Patentinhaber: LANCASTER GROUP GmbH, 55116 Mainz (DE)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: DE9702935
(87) Internationale Veröffentlichungsnummer: WO9826755

(56) Entgegenhaltungen:
- EP-A- 0 565 495
- WO-A-96/17588
- FR-A- 2 715 070
- DWECK A: "TREASURE CHEST OF THE DEEP" SOAP PERFUMERY AND COSMETICS, Bd. 64, Nr. 6, 1.Juni 1991, Seiten 40-41, 43, XP000227206
- DATABASE WPI Section Ch, Week 9432 Derwent Publications Ltd., London, GB; Class A96, AN 94-261706 XP002057711 & RU 2 006 223 C (YUNITEKS RES PRODN FIRM) , 30.Januar 1994
- DATABASE WPI Section Ch, Week 8816 Derwent Publications Ltd., London, GB; Class D21, AN 88-108775 XP002057712 & JP 63 057 510 A (SHISEIDO CO LTD) , 12.März 1988

## Beschreibung

Die Erfindung betrifft ein kosmetisches Reinigungs- und Pflegepräparat, das insbesondere für überfettete Haut geeignet ist, die zusätzlich oftmals auch entzündete Stellen oder Bereiche hat, wie beispielsweise eine pubertäre oder eine besonderen Umweltnoxen ausgesetzte Haut.

Es sind bereits eine Reihe von kosmetischen Mitteln auf dem Markt vorhanden oder vorgeschlagen worden, die besonders für problematische Haut mit übermäßiger Fett/Talgabsonderung und auch entzündlichen Stellen oder Bereichen geeignet ist. Aus der EP-A-721 775 ist eine Kombination von a) Salicylsäure und/oder einer α-Hydroxycarbonsäure und/oder einer α-Ketocarbonsäure, b) einem Glycerinmonocarbonsäuremonoester und c) gegebenenfalls Retinol oder einem Retinolderivat bekannt. Die EP-A-728 474 beschreibt die Verwendung einer Verbindung, die wenigstens eine nicht vollständig neutralisierte Sulfonsäurefunktion aufweist, als Anti-Akne-Wirkstoff bei der Herstellung topischer Zusammensetzungen.

Aus "Parfümerie und Kosmetik", Nr. 12/1997, S. 776-779 ist bekannt, daß Phlorogine als grundlegende Substanz einer Meeresalge den Überschuß an Sebum bei Haut und Haar bekämpft.

Die JP 61-286 317 A, referiert in Patent Abstracts of Japan 1987, Vol. 11, No. 150, beschreibt den Einsatz eines wäßrigen Extraktes von Glycyrrhiza glabra in Bademitteln.

Als Hautpflegemittel wird in der JP 6-256 140 A, referiert in Patent Abstracts of Japan 1994, Vol. 18 No. 651, eine Kombination von Glycyrrhetinsäurederivaten und einem wasserlöslichen UV-Absorptionsmittel beschrieben.

Die mit auf dem Markt befindlichen Präparaten oder vorgeschlagenen Zusammensetzungen erreichbaren Wirkungen sind jedoch nicht immer befriedigend.

Aufgabe der Erfindung ist die Bereitstellung neuer kosmetischer Reinigungs- und Pflegepräparate, die speziell für fettige und entzündliche Haut, insbesondere pubertäre Haut, mit hoher Wirksamkeit geeignet sind.

Erfindungsgemäß bereitgestellt wird ein kosmetisches Reinigungs- und Pflegepräparat, das 0,25 bis 5 Gew-% einer Kombination enthält, bestehend aus
5 bis 40 Gew-% eines Extraktes der Alge Laminaria saccharina; 10 bis 60 Gew-% eines Wurzelextraktes von Lilium candidum;
10 bis 50 Gew-% Glycyrrhetinsäure als natürlicher Wurzelextrakt von Glycyrrhiza glabra; und
das Präparat weiterhin 99,75 bis 95 Gew-% kosmetische Hilfs-und Trägerstoffe, gegebenenfalls im Gemisch mit weiteren Wirkstoffen, enthält.

Aus Untersuchungen ist bekannt, daß zum Beispiel ein Extrakt von Laminaria saccharina das Enzym Lipase, eine Hydrolase, die spezifisch Triglyceride in Glycerin und Fettsäuren spaltet, inhibiert und dadurch die Bildung von freien Fettsäuren und auch von Prostaglandinen verhindert. Allerdings inhibieren Zusätze unter 0,8 % des Extraktes weniger als 20 % der Lipase und sind somit als nicht sehr wirksam anzusehen. Deshalb ist ein Zusatz von wenigstens 1 % dieses Extraktes für eine wirksame Inhibierung von ca. 30 % Lipase vorgeschlagen worden.

Überraschenderweise wurde gefunden, daß eine Kombination der obigen Bestandteile mit einem Gehalt an Laminaria saccharina, der wesentlich unter 0,8 Gew-% liegt, bereits einen sebostatischen Effekt von etwa 30 % und mehr erreicht, so daß eine nicht vorhersehbare Wirkungssteigerung (Synergismus) vorliegt. Mit einem Zusatz von 2 % der Alge, bezogen auf die Gesamtmasse des Präparates, tritt eine Lipase-Inhibierung von etwa 80 % ein. Dies ließ sich durch Fettsäurebestimmungen ermitteln.

Es war weiterhin nicht vorherzusehen, daß das neue Präparat bei gesunder Haut nicht in die hauteigene Mikroflora eingreift und daß die volle Aktivität der beiden anderen Kombinationsbestandteile vollständig erhalten bleibt.

Der Wurzelextrakt von Lilium candidum wirkt normalerweise tonisierend und adstringierend und trägt im vorliegenden Fall auch zur Verstärkung der sebostatischen Wirkung bei.

Von Glycyrrethinsäure ist eine fördernde Wirkung bei der Wundheilung und eine Wirkung gegen Hautjucken bekannt, und sie wurde bisher auch schon bei allergischen Reaktionen, rauher oder spröder Haut in Gesichtswässern und Cremes eingesetzt. Sie trägt im vorliegenden Fall überraschenderweise auch zur Verstärkung der sebostatischen Wirkung bei.

Als weiteren Wirkstoff kann das Präparat vorteilhaft Kaolin gemäß WO96/17588 enthalten, der mit sphärischen TiO₂- oder SiO₂-Teilchen mit einer Teilchengröße <5 *µ*m modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 6 Gew-% haben, bezogen auf die Gesamtmenge des Präparates.

Das Präparat kann als weiteren Wirkstoff einen Blütenextrakt von Matricaria recutita enthalten mit einem Anteil von 0,1 bis 1 Gew-% hat, bezogen auf die Gesamtmenge des Präparates. Dieser pflanzliche Extrakt ist in vielen kosmetischen und dermatologischen Mitteln enthalten und wirkt insbesondere über seinen Bisabologehalt entzündungswidrig auf der Haut.

Das neue Präparat eignet sich speziell für fettige und entzündliche Haut, insbesondere pubertäre Haut, und zeigt auf solchen problematischen Hautbereichen eine hohe Wirksamkeit. Die Fettabsonderung wird deutlich vermindert und gleichzeitig werden Entzündungsherde in kurzer Zeit beseitigt oder wenigstens erkennbar zurückgedrängt. Eine Erhöhung der Konzentration der drei erfindungswesentlichen Kombinationsbestandteile ist möglich und führt zu einer weiteren Verbesserung der Wirkung, die jedoch nicht mehr so ausgeprägt ist, wie bei den vorliegenden Konzentrationen.

Das erfindungsgemäße Präparat enthält weiterhin kosmetische Hilfs- und Trägerstoffe, wie sie üblicherweise in solchen Zubereitungen verwendet werden, z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Polymere, Copolymere, Emulgatoren.

Der pH-Wert des Präparates kann vorteilhaft im sauren Bereich eingestellt werden, wobei ein pH von 5 bis 6 bevorzugt ist.

Die Herstellung erfolgt, indem zuerst der Komplex durch Verrühren bei 15 bis 35 °C von 5 - 40 Gew-% der Alge, 10-60 Gew-% Wurzelextrakt und 10-50 Gew-% Glycyrrhetinsäure hergestellt wird. Zusätzlich können zur Erhöhung des Effektes entsprechende Mengen an modifiziertem Kaolin (gemäß WO96/17588) und Kamillenblütenextrakt unter Rühren hinzugesetzt werden.

Es ist auch möglich, das Präparat in einer zweiphasigen Form herzustellen, beispielsweise als Zweiphasen-Tonisierungsmittel, indem bei Raumtemperatur die Phasen unter Rühren miteinander vermischt werden.

Die Erfindung soll nachstehend durch Beispiele näher erläutert werden. Alle Angaben erfolgen in Gewichtsprozent.
Die auf 100 % ergänzte Wassermenge bezieht sich auf 100 % der gesamtzusammensetzung.

### Beispiel 1 Gesichtsmaske

| **Phase A** | |
|---|---|
| Copolymer | 1,5 |
| Carbomer | 0,5 |
| Propylenglycol | 1,0 |
| Glycerin | 1,5 |
| Kaolin normal | 25,0 |
| Kaolin modifiziert | 5,0 |
| Wasser | ad 100 |

| **Phase B** | |
|---|---|
| Jojobaöl | 2,0 |
| Babassuöl | 2,0 |
| Cetearylalkohol | 2,5 |

| **Phase C** | |
|---|---|
| TEA (Triethanolamin) | 0,8 |

| **Phase D** | |
|---|---|
| Konservierungsmittel | 0,4 |
| Parfüm | 0,5 |

| **Phase E** | |
|---|---|
| Laminaria saccharina-Extrakt | 40 |
| Lilium candidum-Extrakt | 45 |
| Glycyrrhetinsäure | 10 |
| Matricaria recutita-Extrakt | 5 |

Die Phasen A und B wurden separat auf etwa 60 bis 65 °C erwärmt und unter Rühren miteinander vermischt. Danach erfolgte die Zugabe der Phase C und Abkühlung des Gemisches auf 40 °C. Anschließend wurde die Phase C untergerührt. Dann wurden 2,5 Gew-% der Phase E, bezogen auf die Gesamtmasse der Maske, mit dem auf maximal 35 °C abgekühlten Gemisch der anderen Phasen homogen verrührt.

### Beispiel 2 Creme

| **Phase A** | |
|---|---|
| Carbomer | 0,8 |
| Propylenglycol | 2,5 |
| Glycerin | 2,5 |
| Wasser | ad 100 |

| **Phase B** | |
|---|---|
| Jojobaöl | 2,5 |
| Calendulaöl | 1,5 |
| Palmöl | 1,5 |
| Cetearylalkohol | 1,5 |

| **Phase C** | |
|---|---|
| TEA (Triethanolamin) | 0,5 |

| **Phase D** | |
|---|---|
| Aloe Vera | 2 |
| Quittenextrakt | 1 |

| **Phase E** | |
|---|---|
| Laminaria saccharina-Extrakt | 34 |
| Lilium candidum-Extrakt | 25 |
| Glycyrrhetinsäure | 25 |
| Matricaria recutita-Extrakt | 16 |

Die Vermischung erfolgte wie im Beispiel 1, wobei zusätzlich noch 1,5 % modifiziertes Kaolin hinzugegeben wurden und der Anteil der Phase E an der Gesamtzusammensetzung 5 % betrug.

### Beispiel 3 Zweiphasen-Tonisierungsmittel

| **Phase A** | |
|---|---|
| Ethanol | 7 |
| Propylenglycol | 1 |
| Glycerin | 2 |
| Konservierungsmittel | 0,4 |
| modifiziertes Kaolin | 10,0 |
| Wasser | ad 100 |

| **Phase B** | |
|---|---|
| Laminaria saccharina-Extrakt | 20 |
| Lilium candidum-Extrakt | 40 |
| Glycyrrhetinsäure | 25 |
| Matricaria recutita-Extrakt | 5 |

Die Phasen A und B wurden bei Raumtemperatur miteinander vermischt, wobei der Anteil der Phase B an der Gesamtzusammensetzung 1/0 % betrug.

### Beispiel 4 Reinigungsgel

| **Phase A** | |
|---|---|
| Carbomer | 1,5 |
| Propylenglycol | 1,0 |
| Glycerin | 1,0 |
| Wasser | ad 100 |

| **Phase B** | |
|---|---|
| TEA (Triethanolamin) | 1,5 |

| **Phase C** | |
|---|---|
| Ginsengextrakt | 1,0 |
| Parfüm | 0,2 |
| Konservierungsmittel | 0,3 |
| Tegobetain | 1,5 |

| **Phase D** | |
|---|---|
| Laminaria saccharina-Extrakt | 35 |
| Lilium candidum-Extrakt | 22 |
| Glycyrrhetinsäure | 33 |
| Matricaria recutita-Extrakt | 10 |

Die Vermischung erfolgte wie im Beispiel 1, wobei der Anteil der Phase D an der Gesamtzusammensetzung 3,8 % betrug.

## Patentansprüche

1. Kosmetisches Reinigungs- und Pflegepräparat, **dadurch gekennzeichnet, daß** es
(a) 0,25 bis 5 Gew-% einer Wirkstoffkombination aus
5 bis 40 Gew-% eines Extraktes der Alge Laminaria saccharina;
10 bis 60 Gew-% eines Wurzelextraktes von Lilium candidum;
10 bis 50 Gew-% Glycyrrhetinsäure als natürlicher Extrakt von Glycyrrhiza glabra; und
(b) 99,75 bis 95 Gew-% kosmetische Hilfs-und Trägerstoffe enthält.

2. Präparat nach Anspruch 1, **dadurch gekennzeichnet, daß** es weitere Wirkstoffe enthält.

3. Präparat nach Anspruch 2, **dadurch gekennzeichnet, daß** als weiterer Wirkstoff ein mit sphärischen TiO₂- oder SiO₂-Teilchen mit einer Teilchengröße von <5 *µ*m modifizierter Kaolin enthalten ist, wobei die sphärischen Teilchen einen Anteil an der Kaolinmischung von 0,5 bis 10 Gew-% haben.

4. Präparat nach einem der Ansprüche 1 bis 3, **dadurch gekennzeichnet, daß** der modifizierte Kaolin einen Anteil von 0,1 bis 1 Gew-% hat, bezogen auf die Gesamtmenge des Präparates.

5. Präparat nach Anspruch 1 oder 2, **dadurch gekennzeichnet, daß** als weiterer Wirkstoff ein Blütenextrakt von Matricaria recutita enthalten ist, wobei der Gehalt 0,1 bis 1 Gew-%, bezogen auf die Gesamtmenge des Präparates beträgt.

6. Präparat nach einem der Ansprüche 1 bis 5, **dadurch gekennzeichnet, daß** es 0,5 bis 4 Gew-% der Wirkstoffkombination (a) enthält.

7. Präparat nach einem der Ansprüche 1 bis 5, **dadurch gekennzeichnet, daß** es in der Wirkstoffkombination 5 bis 20 Gew-% eines Extraktes der Alge Laminaria saccharina enthält.

## Claims

1. A cosmetic cleansing and skin care preparation comprising
(a) 0.25 to 5 wt% of an active ingredient combination of
5 to 40 wt% of an extract of Laminaria saccharina alga;
10 to 60 wt% of a root extract of Lilium candidum;
10 to 50 wt% glycyrrhetic acid as a natural extract of Glycyrriza glabra; and
(b) 99.75 to 95 wt% cosmetic additives and vehicles.

2. A preparation according to Claim 1, wherein additional active ingredients are also included.

3. A preparation according to Claim 2, wherein a kaolin modified with spherical particles of TiO₂ or SiO₂ with a particle size of <5 µm is also included as an additional active ingredient, with the spherical particles being present in the amount of 0.5 to 10 wt% of the kaolin mixture.

4. A preparation according to one of Claims 1 to 3, wherein the modified kaolin amounts to 0.1 to 1 wt%, based on the total weight of the preparation.

5. A preparation according to Claim 1 or 2, wherein a floral extract of Matricaria recutita is used as an additional active ingredient, the amount being 0.1 to 1 wt%, based on the total weight the preparation.

6. A preparation according to one of Claims 1 to 5, comprising 0.5 to 4 wt% of active ingredient combination (a).

7. A preparation according to one of Claims 1 to 5, comprising 5 to 20 wt% of an extract of the Laminaria saccharina alga in the active ingredient combination.

## Revendications

1. Préparation cosmétique de toilette et de soins, **caractérisée en ce qu'**elle contient :
(a) 0,25 à 5% en poids d'une combinaison de principes actifs comprenant :
5 à 40% en poids d'un extrait de l'algue *Laminaria saccharina* ;
10 à 60% en poids d'un extrait de racine de *Lilium candidum* ;
10 à 50% en poids d'acide glycyrrhétique sous la forme d'extrait naturel de *Glycyrrhiza glabra* ; et
(b) 99,75 à 95% en poids d'adjuvants et véhicules cosmétiques.

2. Préparation selon la revendication 1, **caractérisée en ce qu'**elle contient d'autres principes actifs.

3. Préparation selon la revendication 2, **caractérisée en ce qu'**elle contient comme autre principe actif un kaolin modifié avec des particules sphériques de TiO₂ ou de SiO₂ d'une granulométrie < 5 *µ*m, la proportion des particules sphériques dans le mélange de kaolin allant de 0,5 à 10% en poids.

4. Préparation selon l'une des revendications 1 à 3, **caractérisée en ce que** la proportion du kaolin modifié est de 0,1 à 1% en poids, ramenée à la quantité totale de la préparation.

5. Préparation selon la revendication 1 ou la revendication 2, **caractérisée en ce qu'**elle contient comme autre principe actif un extrait de fleurs de *Matricaria recutita,* la teneur étant de 0,1 à 1% en poids, ramenée à la quantité totale de la préparation.

6. Préparation selon l'une des revendications 1 à 5, **caractérisée en ce qu'**elle contient de 0,5 à 4% en poids de la combinaison de principes actifs (a).

7. Préparation selon l'une des revendications 1 à 5, **caractérisée en ce qu'**elle contient de 5 à 20% en poids d'un extrait de l'algue *Laminaria saccharina* dans la combinaison de principes actifs.
